# EUROPEAN PATENT SPECIFICATION

(11) **EP 4 015 014 B1**
(45) Date of publication and mention of the grant of the patent: **04.10.2023**
(21) Application number: 21214982.7
(22) Date of filing: 16.12.2021
(51) Int. Cl.: A61M 1/16, A61M 1/34

(54) **BLOOD FILTERING DEVICE, PARTICULARLY FOR HEMODIALYSIS AND/OR HAEMOFILTRATION APPARATUSES**
BLUTFILTRIERUNGSVORRICHTUNG, INSBESONDERE FÜR HÄMODIALYSE- UND/ODER HÄMOFILTRATIONSEINRICHTUNGEN
DISPOSITIF DE FILTRAGE DE SANG, EN PARTICULIER POUR APPAREILS D'HÉMODIALYSE ET/OU D'HÉMOFILTRATION

(30) Priority: 18.12.2020 IT 202000031391
(43) Date of publication of application: 22.06.2022
(73) Proprietor: Fluid-O-Tech S.r.l., 20094 Corsico (MI) (IT)
(72) Inventor: TESSICINI, Fabrizio, 40026 Imola (BO) (IT); NORGIA, Michele, 20134 Milano (MI) (IT); ANDREIS, Diego, 20145 Milano (MI) (IT)
(74) Representative: Iannone, Carlo Luigi

(56) References cited:
- EP-A1- 2 790 008
- US-A1- 2012 004 865
- US-A1- 2019 293 543
- US-A1- 2020 030 514

## Description

The present invention relates to a blood filtering device, particularly for hemodialysis and/or haemofiltration apparatuses.

As is well known, there are many medical procedures, mostly therapeutic, during which a patient's blood is subjected to filtration to remove unwanted substances present in the blood or to separate certain components of the whole blood.

The two main mechanisms with which it is possible to purify the blood and thus remove substances and/or components that need to be removed are the hemodialysis, which mainly uses the diffusion principle, and the haemofiltration, which uses the convection principle.

In therapeutic hemodialysis treatments, the blood is taken from the patient, made to flow through a dialyzer filter inside which it comes into contact with a semi-permeable membrane that allows the selective passage, mainly by osmotic diffusion, of the toxic substances to be removed from the blood, and then it is returned to the patient.

In therapeutic haemofiltration treatments, such as therapeutic apheresis, it is provided for the blood taken from a patient in extracorporeal circulation to be filtered through a membrane that separates a specific blood component by convection thanks to a pressure difference, before being reintroduced into the patient, and if necessary supplemented with a solution compatible with the patient's own blood as a replacement for the removed component.

There are also so-called therapeutic haemodiafiltration treatments in which the two mechanisms of dialysis and filtration are adopted simultaneously.

However, the blood filtering devices used in hemodialysis and in haemofiltration are not free from drawbacks, one of which is that the incorrect functioning thereof can have very serious consequences for the patient undergoing the therapeutic treatment.

In addition, the blood filtering devices commonly used in the clinical setting do not allow a real-time monitoring of the performance of the filter itself, not only to ensure its integrity, but also to make sure that the therapeutic treatment is taking place effectively.

To date, the only check that, according to current clinical protocols, is carried out on hemodialysis and haemofiltration apparatuses is to detect traces of blood in the filtrate fraction and/or in the dialysis fluid coming out of the filter, as the presence of blood indicates a rupture of the filter membrane. With regard to this, there are in fact international standards, such as the IEC 60601-2-1 standard, which define a maximum blood threshold, expressed in terms of flow rate, which can be detected at the filter outlet. Beyond this threshold, an alarm is activated to interrupt the therapeutic treatment and restore the filter.

This threshold, in the case of hemodialysis, is currently equal to a flow rate of 0.35 ml/min of blood in flows of dialysis fluid having flow rates of 800 ml/min. This corresponds to a blood volume of 218µL in 500mL of dialysis fluid, i.e. a dilution ratio of 1 in 2285 .

This check is currently carried out using optical sensors (spectrophotometers) to detect blood loss, so-called "BLD" sensors, from the acronym "BLOOD LEAK DETECTOR", which are placed at the outlet of the hemodialysis or haemofiltration filter.

One of the main drawbacks that afflict BLD sensors of the known type consists in the fact they have a sensitivity that, although compatible with the minimum requirements imposed by the aforesaid international standards, is relatively low. In fact, these sensors are activated only when significant amounts of blood pass, as there must be a perceptible change in the absorption value of the effluent under examination at the characteristic wavelengths of the haemoglobin.

Another drawback consists in the fact that BLD sensors of the known type often generate false positive results, as the presence of traces of blood in the filtrate fraction and/or in the dialysis fluid is mistaken for the presence of other substances that cause colour changes in the effluent, such as bilirubin. Similarly, BLD sensors of the known type do not allow to distinguish whether the presence of traces of blood leaving the filter is due to a phenomenon of haemolysis, which is often independent of the integrity of the filter.

Furthermore, BLD sensors often malfunction when exposed to inadequate ambient lighting conditions.

As mentioned above, another drawback of the hemodialysis and haemofiltration apparatuses commonly used in the clinical setting consists in the fact that a real-time analysis of parameters indicative of the effectiveness of the therapeutic treatment to which the patient is subjected is not generally provided.

For example, in the case of hemodialysis, the effectiveness of dialysis treatment depends on the extent to which toxins are removed from blood. In this respect, blood urea concentration is commonly used as a measure of blood toxicity.

Currently, the standard clinical procedure for monitoring hemodialysis therapy is to collect pre- and post-dialysis blood samples from time to time, e.g. once a month during a dialysis session, which are analysed to quantify the urea content in a clinical laboratory. It is therefore clear that such an occasional analysis, in addition to being particularly laborious and costly, is not indicative of the effectiveness of the single hemodialysis treatment.

Similar considerations can also be made with regard to haemofiltration therapies.

Relevant prior art is for instance disclosed in documents US2020/030514 A1, EP2790008 A1, US2019/293543 A1 and US2012/004865 A1.

The main task of the present invention consists in realising a blood filtering device, particularly for hemodialysis and/or haemofiltration apparatuses, which obviates the drawbacks and overcomes the limits of the prior art by allowing the integrity and performance of the filter itself to be monitored in real time with high sensitivity and accuracy.

Within the scope of this task, one object of the present invention is to realise a blood filtering device which detects the presence, even minimal, of traces of blood leaving the filter, minimising false positive results.

A further object of the invention consists in realising a blood filtering device which is capable of giving the greatest assurances of reliability and safety in use.

Another object of the invention consists in realising a blood filtering device that is easy to manufacture and economically competitive if compared to the prior art, as well as easy to integrate into commonly used hemodialysis and/or haemofiltration apparatuses.

The aforementioned task, as well as the aforementioned objects and others which will become clearer hereinafter, are achieved by a blood filtering device, particularly for hemodialysis and/or hemofiltration apparatuses as claimed in claim 1.

Other features are provided in the dependent claims.

Further characteristics and advantages will become clearer from the description of two preferred, but not exclusive, embodiments of a blood filtering device, illustrated by way of non-limiting example with the aid of the attached drawings in which:

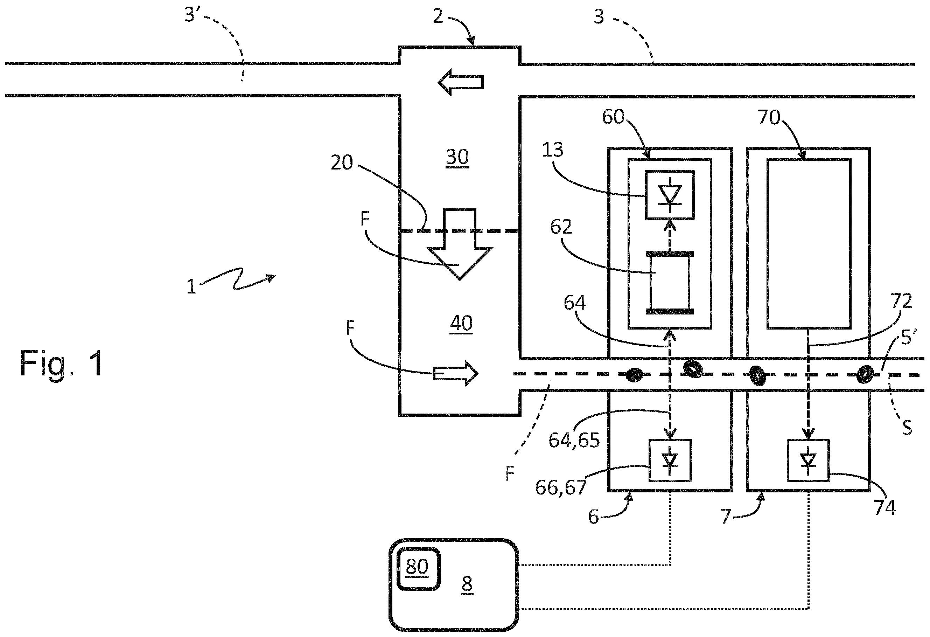

Figure 1 is a schematic representation of a first embodiment of a blood filtering device, according to the invention, particularly for haemofiltration apparatuses;
Figure 2 is a schematic representation of a second embodiment of a blood filtering device, according to the invention, particularly for hemodialysis apparatuses;

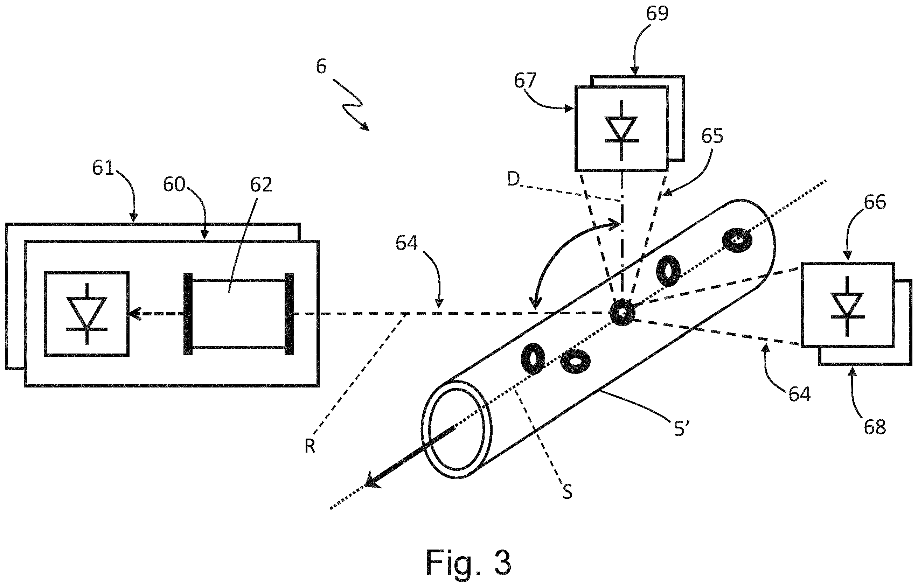

Figure 3 schematically illustrates the operating principle of a sensor at the outlet of the blood filtering device, according to the invention;
Figure 4 is a schematic representation of a hemodialysis apparatus, comprising a blood filtering device, according to the invention;
Figure 5 is a representative graph of the operation of the sensor provided at the outlet of the blood filtering device, according to the invention;
Figure 6 is a graph representative of an enlarged portion of the graph of Figure 5;
Figure 7 is a further graph representative of the operation of the sensor provided at the outlet of the blood filtering device, according to the invention.

With reference to the above-mentioned figures, the blood filtering device, particularly for hemodialysis and/or haemofiltration apparatuses, globally indicated with reference number 1, comprises a filter 2 comprising a first compartment 30, adapted to allow the passage of blood 3, 3', and a second compartment 40, separated from the first compartment 30 by means of a membrane 20 adapted to allow the passage of a filtered fraction F from the first compartment 30 to the second compartment 40. The filtering device 1 further comprises an outlet conduit 5' where, at least, the filtered fraction F leaving the filter 2 is collected. The filtered fraction F flows along said outlet conduit 5' along a flow direction S.

As shown in the accompanying figures, the filtering device 1 receives at its inlet a flow of blood to be filtered, indicated with 3. Inside the filter 2, a filtered fraction F passes from the first compartment 30, through the membrane 20, to the second compartment 40, being separated from the incoming blood 3 and being collected in the outlet conduit 5' of the filtering device 1. Reference 3' indicates the filtered blood leaving the filtering device 1.

According to the invention, the filtering device 1 comprises a first sensor 6 comprising:
- at least one semiconductor laser source 60, 61 comprising a laser cavity 62 and adapted to generate a laser light beam 64 which strikes the outlet conduit 5' along an irradiation direction R incident to the flow direction S;
- at least one front photodiode 66, 68 placed along the irradiation direction R on a side opposite to the semiconductor laser source 60, 61 with respect to the outlet conduit 5',
- at least one lateral photodiode 67, 69 placed along a diffusion direction D substantially orthogonal to the irradiation direction R.

At least in correspondence of the semiconductor laser source 60, 61, of the at least one front photodiode 66, 68 and of the at least one lateral photodiode 67, 69, the outlet conduit 5' is transparent to the laser light beam 64.

The at least one front photodiode 66, 68 generates a first electrical signal dependent on the modulation of the power of the laser light beam 64 operated, according to a retro-injection interferometry effect (so-called "self-mixing interferometry" effect), by suspended particles present within the filtered fraction F and moving along the outlet conduit 5'.

The at least one lateral photodiode 67, 69 instead generates a second electrical signal depending on the part 65 of the laser light beam 64 which is diffused by the filtered fraction F along substantially the diffusion direction D.

Finally, the filtering device 1 comprises a processing and control unit 8 programmed to process the first electrical signal, generated by the at least one front photodiode 66, 68, and the second electrical signal, generated by the at least one lateral photodiode 67, 69, and to generate, on the basis of said two electrical signals, a signal indicative at least of the quantity of the suspended particles moving along the outlet conduit 5'.

Advantageously, from the combination of the electrical signals obtainable due to the presence of the two front photodiodes 66 and lateral photodiodes 67 it is possible to generate a signal representative of the presence of blood in the filtered fraction F in a very wide measuring range, since the front photodiode 66, operating in self-mix, is also sensitive to the passage of the single red blood cell inside the filtrate fraction F, while the lateral photodiode 67 is able to operate correctly when the quantity of blood, i.e. red blood cells, inside the filtrate fraction F becomes preponderant. In this situation, the front photodiode 66 goes into saturation while the lateral photodiode 67 continues to detect the radiation diffused by the red blood cells.

Advantageously, if the membrane 20 of the filter 2 is adapted to prevent the passage of particles such as red blood cells, the possibility of detecting their presence and quantity in the filtrate fraction F provides direct indications about the integrity of the filter 2 itself.

Advantageously, the processing and control unit 8 is programmed to generate an alarm signal based on the signal indicative at least of the quantity of suspended particles moving along the outlet conduit 5', when this signal exceeds a predefined threshold value.

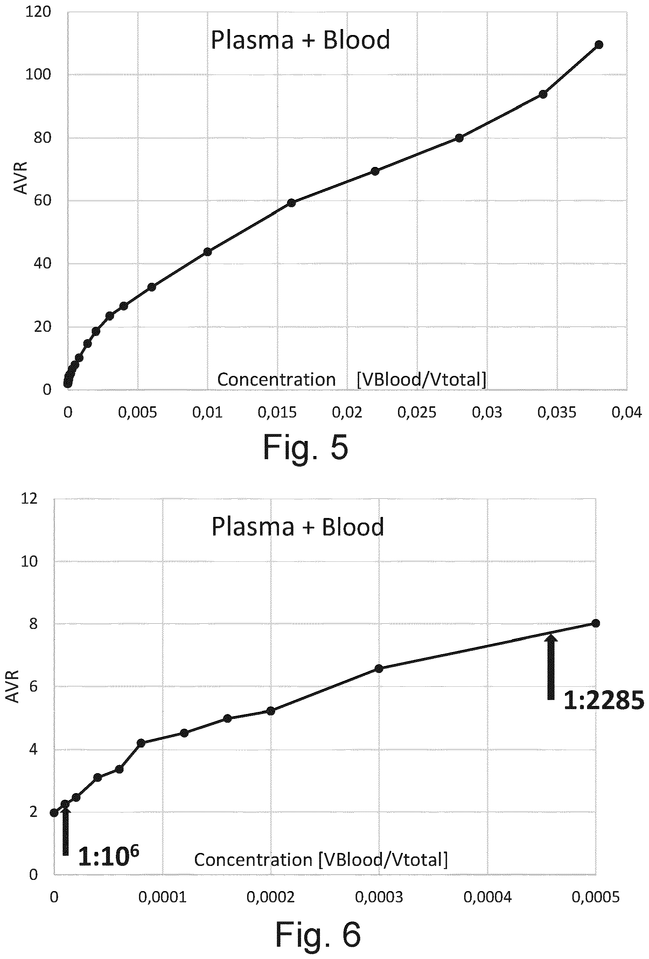

In this respect, Figure 5 shows a graph illustrating the operation of the sensor 6 as the concentration of blood inside the plasma varies. The "AVR" signal, indicative of the quantity of suspended particles moving along the conduit, shows that sensor 6 is capable of detecting near-zero blood volumes up to blood volumes equal to 4% of the total volume of the liquid. The experiment whose results are shown in the graph of Figure 5 simulates the operation of the sensor 6 at the output of a plasmapheresis filter, in which the sensor 6 detects the presence of blood, that is red blood cells, in the plasma to ascertain that the filter is operating correctly by separating only the plasma component from the whole blood.

The experiment was carried out by adding known gradually increasing quantities of blood to a base liquid consisting of plasma.

The graph shown in Figure 6 is an enlargement of the graph of Figure 5 and shows how the sensor 6 is able to detect traces of blood in the plasma with concentrations equal to 1 PPM (1:10⁶). For the sake of completeness, the graph in Figure 6 also indicates the value of the "AVR" signal in correspondence of blood concentrations corresponding to the minimum detectable according to the international standard IEC 60601 mentioned in the introduction (i.e., one part of blood on 2285 parts of liquid, 1:2285).

The "AVR" signal shown on the ordinate in the graphs of figures 5 and 6 is a dimensionless signal obtained by calculating the ratio between the root mean square (RMS) of the electrical signal generated by the lateral photodiode 67 and the average value of the electrical signal generated by the front photodiode 66.

Advantageously, starting from the combination of the electrical signals obtainable thanks to the presence of the aforesaid two front 66 and lateral 67 photodiodes, it is possible to implement different types of processing and calculations to obtain a signal indicative of the presence of suspended particles moving along the outlet conduit 5' of the filtering device 1.

The calculation proposed above for obtaining the "AVR" signal shown in the graphs in Figures 5 and 6 was chosen because it is capable of continuously generating a signal indicative of the presence of blood in the plasma within a very wide range of values.

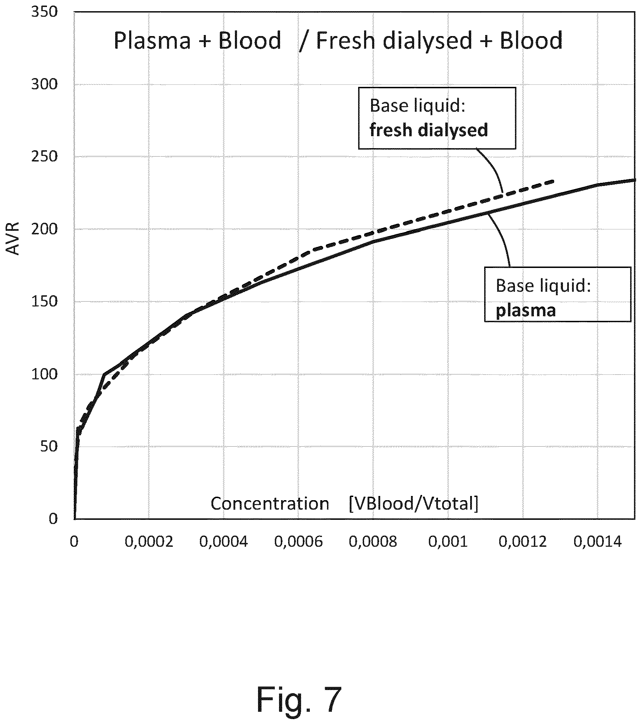

The graph illustrated in Figure 7 shows the comparison between the capacity of the sensor 6 to detect blood added in known gradually increasing quantities in a base liquid consisting of plasma and in a base liquid consisting of fresh dialysis liquid, used at the inlet to the hemodialysis apparatuses.

As is evident from the comparison of the two trends shown in the graph in Figure 7, the sensor 6 is able to correctly detect the presence of blood without being affected by the specific properties of the base liquid in which the blood is present.

As also explained below, other types of processing and calculations can be implemented, starting from the electrical signals generated by the front 66 and lateral 67 photodiodes, on the basis of different applications and on the basis of different substances and/or components whose presence in the liquid leaving the outlet conduit 5' is to be verified.

Advantageously, through the analysis of the so-called "self-mix" signal generated by the front photodiode 66 it is possible to have a very sensitive measurement of the quantity of particles present in the filtrate fraction F, as it is also possible to detect the presence of even very small single particles, such as red blood cells, which otherwise could not be detected by a normal photodiode in any other way. Therefore, as mentioned above, the filtering device 1 is extremely sensitive in detecting minimal traces of blood due to the presence of red blood cells in the filtrate fraction F.

The high sensitivity is also combined with a high accuracy in detecting the presence of red blood cells. In fact, thanks to the fact that the filtering device 1 takes into account both the self-mix signal detected by the front photodiode 66 and the signal related to the diffusion of the laser radiation detected by the lateral photodiode 67, it is possible to reduce the undesired effects due to the responses of the photodiodes in the presence of ambient light.

In a practical example, the sensor 6 can easily distinguish the presence of red blood cells in the filtrate fraction F, e.g. due to a rupture in the filter 2, from the presence of haemoglobin dissolved in the blood as a result of a haemolysis phenomenon independent of the integrity of the filter 2.

In the first case, the front photodiode 66, operating in self-mix, will detect the passage of red blood cells, while the lateral photodiode 67 will detect the part of laser radiation diffused by them. In the second case, the front photodiode 66 will not detect any passage of red blood cells, while the lateral photodiode 67 will still detect a laser radiation diffused by the presence of hemoglobin dissolved in the filtrate fraction F.

The expression "flow direction S" means the direction along which the filtrate fraction F flows within the outlet conduit 5', with particular reference to the portion of said conduit 5' which is struck by the laser light beam 64. In the case in which the conduit 5, in the portion thereof which is struck by the laser light beam 64, has a rectilinear course, said flow direction S coincides, or is parallel, with the central axis of the conduit 5. In the case in which the conduit 5' has, precisely in the portion thereof which is struck by the laser light beam 64, a curvilinear course according to a curved line, the expression "flow direction S" means the direction tangent to the curved line near the area of the conduit 5 struck by the laser light beam 64.

The term "incident" means that the flow direction S and the irradiation direction R have a common point, that is, they intersect defining an angle greater than 0° .

Preferably the angle of incidence between the flow direction S and the irradiation direction R is substantially equal to 90°.

Advantageously, the sensor 6 comprises a monitor photodiode 13 arranged upstream of the laser cavity 62, adapted to generate an electrical signal also dependent on the modulation of the power of the laser light beam 64 (so-called self-mix signal). In this case, the processing and control unit 8 is programmed to also process the electrical signal generated by the monitor photodiode 13 to improve the signal-to-noise ratio of the signal indicative of the quantity of suspended particles moving along the outlet conduit 5'.

In fact, the front photodiode 66, 68 and the monitor photodiode 13 both measure the amplitude modulations of the laser light beam 64 induced by the self-mix effect. However, these modulations have opposite signs between them. Therefore, by calculating the difference between the two self-mix signals detected by the front photodiode 66, 68 and by the monitor photodiode 13, a gain of a factor of two is obtained on the amplitude of the self-mix signal, and also a subtraction of all the common disturbances is obtained, such as the noise and the disturbances of the supply of the laser source, as well as the "shot-noises" and the "1/f" noise of the laser itself.

Advantageously, the processing and control unit 8 is programmed to process the first and the second electrical signals to also generate a signal indicative of the type and/or quantity of solutes present in the filtrate fraction F, such as for example urea, hemoglobin, bilirubin.

Advantageously, the processing and control unit 8 comprises a programmable memory 80 configured to receive and store at least one reference signal associated with at least one specific type of solute present in a reference liquid. The processing and control unit 8 is then programmed to generate a signal indicative of the quantity of said type of solute present in the filtrate fraction F on the basis of a comparison with the signal reference associated with said type of solute stored in the programmable memory 80. This comparison can advantageously be made in real time in the processing and control unit 8, so that the signal indicative of the quantity of this type of solute present in the filtrate fraction F can be obtained in real time.

Advantageously, it is also possible to store a plurality of reference signals associated with a plurality of different types of solutes present in a reference liquid, so as to generate a plurality of signals indicative of the quantity of each of these solutes present in the filtrate fraction F.

In essence, it is possible to store, in the programmable memory 80 of the processing and control unit 8, a "fingerprint" of a reference liquid in relation to a plurality of different solutes whose presence in the filtrate fraction F is to be ascertained. In this way, the comparison of the signals generated by the processing and control unit 8 starting from the measurements of the photodiodes 66 and 67 with the aforesaid "fingerprint" of a reference liquid allows to estimate the amount of the solutes of interest in the filtrate fraction F.

Advantageously, the programmable memory 80 is configured to receive and store at least one reference signal associated with at least one specific type of solute present in a reference liquid consisting of the filtrate fraction F obtained by filtering the blood of a patient in a given therapeutic session. In this case, the "fingerprint" is taken on the patient's own filtrate fraction F, to be used in the analysis of subsequent therapeutic sessions in order to evaluate their effectiveness over time.

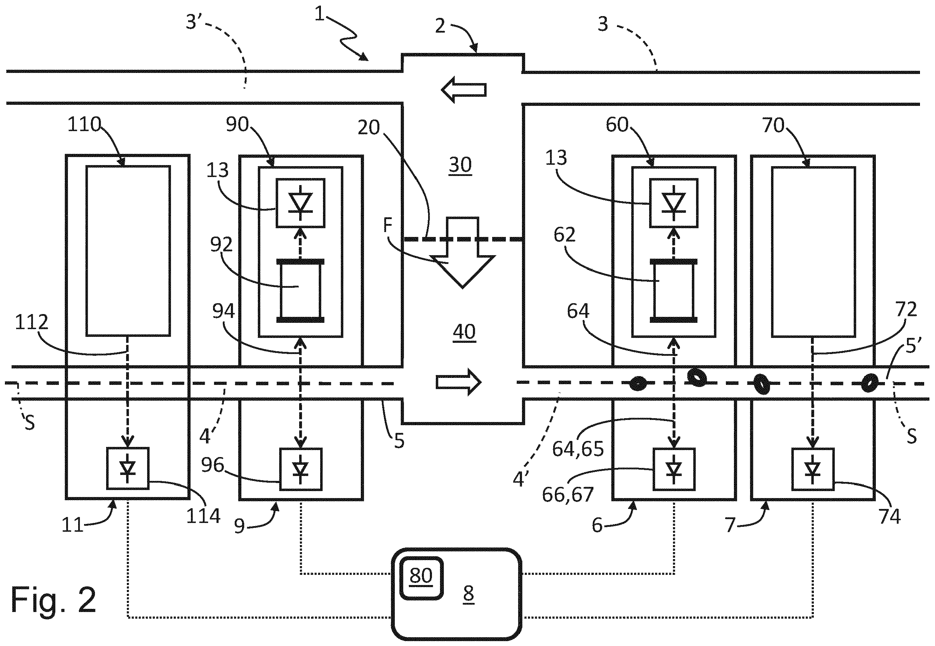

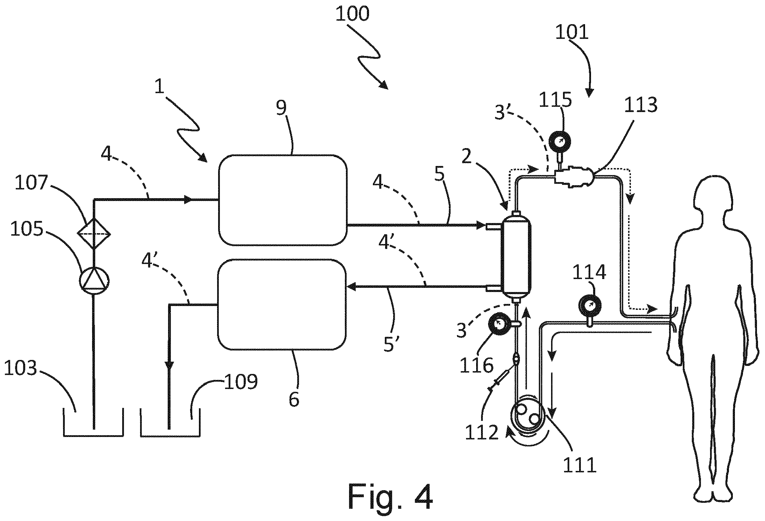

Advantageously, as illustrated in particular in Figure 2 and Figure 4, the blood filtering device 1 may be particularly suitable for hemodialysis procedures. The filtering device 1 may in fact be an integral part of a hemodialysis apparatus 100 as illustrated in Figure 4.

In this case, the filter 2 is a dialyzer filter comprising a first compartment 30, adapted to allow the passage of blood 3, 3', and a second compartment 40, adapted to allow the passage of a dialysis fluid 4, 4'. The first compartment 30 and the second compartment 40 are separated by a semi-permeable membrane 20 which is selective to the crossing of the filtrate fraction F from the blood 3 to the dialysis fluid 4, according to an osmotic phenomenon. In this case the outlet conduit 5' is adapted to collect a mixture 4' of the filtrate fraction F and of the dialysis liquid.

Therefore, the first electrical signal generated by the front photodiode 66, 68 depends on the modulation of the power of the laser light beam 64 operated, according to a retro-injection interferometry effect, by suspended particles present within the mixture 4' of the filtrate fraction F with the dialysis fluid moving along the outlet conduit 5'.

Correspondingly, the second electrical signal generated by the lateral photodiode 67, 69 depends on the part 65 of the laser light beam 64 which is diffused by the mixture 4' of the filtrate fraction F with the dialysis fluid along substantially the diffusion direction D. For convenience, the mixture 4' of the filtrate fraction F with the dialysis fluid will also be referred to simply as "dialysis fluid 4' at the output".

Advantageously, from the combination of the electrical signals obtainable thanks to the presence of the two front 66 and lateral 67 photodiodes, it is possible to generate not only a signal representative of the presence of blood in the dialysis fluid 4' at the output of the filtering device 1, as described above, but also to generate a signal representative of the type and quantity of solutes present in said dialysis fluid 4' .

Advantageously, the processing and control unit 8 is programmed to process the first electrical signal, generated by the front photodiode 66, and the second electrical signal, generated by the lateral photodiode 67, to generate a signal indicative of the quantity of urea present in the mixture 4' of the filtrate fraction F and of the dialysis fluid.

Advantageously, the processing and control unit 8 is programmed to perform an algorithm classifying one or more features of the first electrical signal and one or more features of the second electrical signal and to generate said signal indicative of the quantity of urea present in the mixture 4' of the filtrate fraction F and of the dialysis fluid.

Advantageously, the aforesaid classifying algorithm may be defined starting from automated machine learning techniques, preferably starting from automated learning techniques based on the so-called "random decision forest" classification methods capable of identifying, among the many statistical features of two or more input signals, the main features that allow to robustly estimate a desired output signal, such as precisely a signal indicative of the amount of urea present in the dialysis fluid 4' leaving the filtering device 1.

In particular, it is possible to store in the programmable memory 80 of the processing and control unit 8 a "fingerprint" of the urea consisting of the main statistical features that best describe the presence of urea in a reference fluid, so that the processing and control unit 8 can generate in real time the signal indicative of the quantity of urea present in the dialysis fluid 4' leaving the filtering device 1.

In other words, once the machine learning algorithm has been trained to recognise the presence of urea in a reference fluid, this algorithm can be stored in the programmable memory 80 and be executed by the processing and control unit 8 which, by correlating the two signals generated by the two photodiodes 66 and 67, is able to estimate in real time the amount of urea present in the dialysis fluid 4' and thus to allow the effectiveness, for the patient, of the current dialysis session to be known in real time.

Therefore, starting from the same signals generated by the front photodiode 66 and by the lateral photodiode 67, it is possible to derive different types of information on different properties of the filtrate fraction F and/or of the dialysis liquid 4' leaving the filter 2 through different processing of the aforesaid two signals and performing different statistical calculations.

As indicated, it is possible, for example, to detect the presence and the quantity of blood in the dialysis fluid 4' leaving a hemodialysis filter 2, distinguishing among other things the presence of red blood cells, which may indicate a rupture in the filter 2 itself, from the presence of haemoglobin dissolved in the dialysis fluid 4', which may indicate a haemolysis phenomenon, or even the amount of urea present in the dialysis fluid 4' leaving the filter 2, which provides a real-time indication of the progress of the hemodialysis therapy.

Advantageously, the at least one semiconductor laser source 60, 61 is adapted to generate a laser light beam 64 having a wavelength comprised between 600 and 850 nm, preferably comprised between 750 and 800 nm, and even more preferably equal to about 780 nm. Advantageously, the front 66 and lateral 67 photodiodes are operational at least in a working range compatible with the wavelength of the laser light beam 64.

With a semiconductor laser source 60 that is adapted to generate a laser light beam 64 having a wavelength of about 780 nm it has been possible to obtain information on the presence of blood in the plasma, as illustrated in the graphs shown in Figures 5 and 6. Advantageously, the semiconductor laser source 60 is adapted to emit a laser light beam 64 within the spectral range of absorption of the particle and/or of the solute to be investigated, in this case blood, i.e. an emission in the spectral range of absorption of red blood cells and haemoglobin.

Moreover, with the same experimental set-up, and in particular with a semiconductor laser source 60 generating a laser light beam 64 having a wavelength of about 780 nm, it has been possible to develop a classification algorithm capable of estimating, starting from the combination of the signals generated by the two photodiodes 66 and 67, the amount of urea present in a test liquid.

Advantageously, since the urea absorption spectral range is centred on 280 nm, the selection of a semiconductor laser source 60 generating a laser light beam 64 having a wavelength of about 280 nm is preferable for the purpose of identifying urea in the dialysis fluid 4' leaving the filter 2.

Advantageously, as illustrated in Figures 2 and 4, the filtering device 1 comprises an inlet conduit 5 adapted to convey the dialysis liquid 4 inlet to the dialyzer filter 2. This dialysis fluid 4 flows along the inlet conduit 5 according to a flow direction S.

The filtering device 1 advantageously comprises a second sensor 9 comprising:
- at least one semiconductor laser source 90 comprising a laser cavity 92 and adapted to generate a laser light beam 94 which strikes the inlet conduit 5 along an irradiation direction incident to the flow direction S;
- at least one front photodiode 96 placed along the irradiation direction on a side opposite to said semiconductor laser source 90 with respect to the inlet conduit 5,
- at least one lateral photodiode 97 placed along a diffusion direction D substantially orthogonal to the irradiation direction R.

At least in correspondence of the semiconductor laser source 90, of the at least one front photodiode 96 and of the at least one lateral photodiode 97, the inlet conduit 5 is transparent to the laser light beam 94.

The at least one front photodiode 96 generates an electrical signal dependent on the modulation of the power of the laser light beam 94 operated, according to a retro-injection interferometry effect (so-called "self-mixing interferometry" effect), by suspended particles possibly present within the dialysis fluid 4 and moving along the inlet conduit 5.

On the other hand, the at least one lateral photodiode 97 generates an electrical signal depending on the part of the laser light beam 94 which is diffused by the dialysis liquid 4 along substantially the diffusion direction D.

The processing and control unit 8 is in this case programmed to use the two electrical signals generated by the front photodiode 96 and by the lateral photodiode 97 in subtraction respectively of the two electrical signals detected by the front photodiode 66 and by the lateral photodiode 67 of the first sensor 6 to generate said signal indicative of at least the quantity of said suspended particles moving along the outlet conduit 5' deprived of the disturbances common to the electrical signals of the first sensor 6 and of the second sensor 9.

Advantageously, the second sensor 9 is substantially a replica of the first sensor 6.

In particular, the second sensor 9 may have exactly the same components as the first sensor 6.

In this way, it is further ensured that the signals generated by the first sensor 6 can be used differentially from the signals generated by the second sensor 9 to eliminate all common mode disturbances, such as those of an electrical nature, those due to external ambient lighting conditions, and those due to particular physical/chemical features of the dialysis fluid 4.

Advantageously, the first sensor 6 comprises a first semiconductor laser source 60 and at least a further source 61, 61' that is selectable between:
(i) at least a second semiconductor laser source 61 adapted to generate a laser light beam having a different wavelength with respect to the laser light beam 64 generated by the first semiconductor laser source 60, and
(ii) at least a radiation source 61' adapted to generate a radiation that strikes the outlet conduit 5' along an irradiation direction incident to the flow direction S.

Advantageously, the radiation source 61', such as an LED, emits a radiation having a much wider emission spectrum than the emission spectrum of the laser source 60, which is adapted to emit a coherent radiation beam.

Thus, in a first example, the first sensor 6 comprises a first semiconductor laser source 60 and at least a further radiation source 61', such as an LED.

In this case, the at least one front photodiode 66, 68 generates an electrical signal indicative of the transmittance of the radiation emitted by the radiation source 61' through the filtrate fraction F (or through the dialysis fluid 4' at the output), wherein the transmittance of said radiation depends on the quantity and/or type of solutes present in the filtrate fraction F, while the at least one lateral photodiode 67, 69 generates an electrical signal depending on the part of said radiation which is diffused by the filtrate fraction F (or by the dialysis liquid 4' at the output) along substantially the diffusion direction D.

Advantageously, therefore, with the same set of photodiodes, i.e., with the same front photodiode 66 and with the same lateral photodiode 67, it is possible to operate the first sensor 6 as described above, i.e., also using the self-mix operation, and also as a spectrophotometric sensor.

It is in fact possible, for example, to synchronise the activation of the first laser source 60 in an alternating manner with respect to the activation of the further radiation source 61', so that the same photodiodes 66, 67 are sensitive in an alternating manner in time to the two different types of radiation.

Advantageously, the radiation emitted by the further radiation source 61' may present a spectrum of wavelengths which also include the wavelength of the radiation constituting the laser light beam 64 emitted by the first semiconductor laser source 60. Basically, the radiation emitted by the source 61' and the laser emitted by the laser source 60 can overlap in terms of wavelength values.

Advantageously, it is also possible to provide for a plurality of different radiation sources 61', such as different LEDs, centred on different wavelengths.

Advantageously, therefore, the first sensor 6 integrates, in a compact manner and with a limited number of components, both the possibility of carrying out an interferometric analysis and the possibility of carrying out spectrophotometry.

In a second example, the first sensor 6 comprises at least two semiconductor laser sources 60, 61, wherein a first semiconductor laser source 60 is adapted to generate a laser light beam 64 having a different wavelength with respect to the laser light beam generated by a second semiconductor laser source 61.

Advantageously, the sensor 6 comprises at least two front photodiodes 66, 68 placed along the irradiation direction R on a side respectively opposite to the first semiconductor laser source 60 and to the second semiconductor laser source 61 with respect to the outlet conduit 5', and at least two lateral photodiodes 67, 69 placed along a diffusion direction D substantially orthogonal to the irradiation direction R.

The possibility of providing a plurality of laser sources 60, 61 operating at different wavelengths, and possibly a corresponding plurality of front 66, 68 and lateral 67, 69 photodiodes, makes it possible to improve the sensitivity of the sensor 6 to the detection of specific particles and/or solutes as a function of the relative absorption spectrum.

As mentioned above, a laser source at about 780 nm is preferably used to identify and quantify blood in the filtrate fraction F and can also be used to estimate the amount of urea in the filtrate fraction F or in the dialysis fluid 4' at the output of the filter 2, by means of a classifying algorithm.

Preferably, the possibility of using laser sources 60 and 61 operating at different wavelengths allows to improve the selectivity of the sensor 6 in detecting different particles and/or solutes.

For example, a semiconductor laser source adapted to emit a laser light beam 64 at a wavelength comprised between 200 and 400 nm, preferably comprised between 200 and 300 nm, e.g. equal to about 280 nm, can be used for improving the selectivity of measurement of substances such as the urea present in the dialysis liquid 4' at the output of a dialyzer filter 2.

Basically, the fact of providing a plurality of laser sources 60, 61 operating at different wavelengths, and possibly a plurality of front 66, 68 and lateral 67, 69 photodiodes if a single photodiode does not have an operating range sufficient to cover the overall range of radiations emitted by the different laser sources 60, 61, allows to increase the performance of the sensor 6 making it usable to detect, in a very sensitive manner, the presence and quantity of different types of particles and/or different types of solutes.

Advantageously, the first sensor 6, as well as, similarly, the second sensor 9, may comprise at least one control photodiode 55 adapted to intercept the laser light beam 64 directly emitted by the at least one semiconductor laser source 60 (i.e., adapted to intercept the laser light beam 64 in an area where it does not pass through, or has not yet passed through, the outlet conduit 5'). This control photodiode 55 generates an electrical control signal directly dependent on the laser light beam 64. Advantageously, the processing and control unit 8 is programmed to also process said electrical control signal in order to generate a signal indicative at least of the quantity of suspended particles along the outlet conduit 5', or also a signal indicative of the type and/or the quantity of solutes present in the filtrate fraction F.

Advantageously, the filtering device 1 comprises, in addition to sensors 6 and 9, at least one spectrophotometric sensor 7, which comprises:
- a radiation source 70 adapted to generate a radiation 72 which strikes the outlet conduit 5' along a direction of radiation incident to the flow direction S,
- a photodiode 74 placed along the irradiation direction on a side opposite to the radiation source 70 with respect to the outlet conduit 5'.

This photodiode 74 generates an electrical signal indicative of the transmittance of the radiation through the filtrate fraction F, which transmittance depends on the quantity and/or type of solutes present in the filtrate fraction F.

The processing and control unit 8 is programmed to process the aforesaid electrical signal to generate a signal indicative of the quantity and/or type of solutes present in the filtrate fraction (F).

On the basis of the type of spectrometric sensor 7 used, the filtering device 1 is capable of providing information about solutes of a different type from the types that the sensor 6 is instead capable of detecting, or even redundant information about the same solutes that the sensor 6 is capable of detecting, thus making the analysis of the performance of the filtering device 1 even more robust.

Advantageously, the radiation source 70 of the spectrophotometric sensor 7 is adapted to generate a radiation 72 having a wavelength comprised between 500 nm and 850 nm, wherein the transmittance of the radiation 72 depends on the amount of hemoglobin (and/or bilirubin) present in the filtrate fraction F. The processing and control unit 8 is in this case programmed to process the electrical signal generated by the photodiode 74 so as to generate a signal indicative of the quantity of hemoglobin and/or bilirubin present in the filtrate fraction F.

Advantageously, the radiation source 70 of the spectrophotometric sensor 7 is adapted to generate an ultraviolet radiation 72 or in the Near Infrared (NIR) range, wherein the transmittance of the radiation 72 depends on the amount of urea present in the filtrate fraction F, and in particular in the mixture 4' of the filtrate fraction 4 with the dialysis fluid. The processing and control unit 8 is in this case programmed to process the electrical signal generated by the photodiode 74 so as to generate a signal indicative of the quantity of urea present in the filtrate fraction F.

In addition, a plurality of spectrophotometric sensors 7 placed in correspondence of the outlet conduit 5' can be provided, each configured to detect the presence and the amount of a different type of solute within the same filtrate fraction F.

Advantageously, in the case of a filtering device 1 for hemodialysis apparatuses 100, an inlet conduit 5 is adapted to convey the dialysis fluid 4 inlet to the filter 2 dialyzer, wherein said dialysis fluid 4 flows along the inlet conduit 5 according to a flow direction S. A second spectrophotometric sensor 11 is advantageously present in correspondence of the inlet conduit 5, having technical characteristics corresponding to those of the spectrophotometric sensor 7 described above.

In this case, the second spectrophotometric sensor 11 placed at the inlet of the dialyzer filter 2 allows to generate information useful for a better processing of the information derivable from the spectrophotometric sensor 7 placed at the outlet of the dialyzer filter 2, for example to eliminate common mode disturbances, as well as to generate information related to the characteristics of the dialysis liquid 4 at the inlet of the same filter 2.

Advantageously, the filtering device 1 also comprises one or more of the following sensors:
- a temperature sensor of the filtrate fraction F (or of the dialysis fluid 4' at the output of the device 1);
- a flow rate sensor for the filtrate fraction F (or of the dialysis fluid 4') flowing along the outlet conduit 5';
- a conductivity sensor of the filtrate fraction F (or of the dialysis fluid 4' at the output of the device 1).

The processing and control unit 8 is programmed to also process the signals generated by such sensors to generate a signal indicative of the quantity of suspended particles moving along the outlet conduit 5', as well as the type and/or the amount of solutes present in the filtrate fraction F.

The present invention further relates to a hemodialysis and/or haemofiltration apparatus 100 comprising a blood filtering device 1 as described above.

For example, Figure 4 illustrates a hemodialysis apparatus 100 comprising a filtering device 1 whose dialyzer filter 2 is connected to a circuit for extracorporeal circulation 101 of blood.

As illustrated schematically in Figure 4, the hemodialysis apparatus 100 comprises a reserve 103 of fresh dialysis fluid, which is pumped, by means of the pump 105, possibly in the presence of a filter 107, towards the dialyzer filter 2. The dialysis fluid 4' leaving the dialyzer filter 2 is instead collected in a discharge volume 109, to be disposed of, or to be reused after an appropriate regeneration.

The hemodialysis apparatus 100 then comprises a pump 111, of the peristaltic type, adapted to put part of the patient's blood into extracorporeal circulation, a system for introducing heparin 112 into the blood taken from the patient, a system for removing air 113 possibly present in the blood, before the re-introduction thereof into the patient. There are also provided some devices for detecting and displaying arterial pressure 114 and venous pressure 115, as well as devices 116 for detecting and displaying the flow of blood entering the dialyzer filter 2.

The present invention further relates to a process for detecting suspended particles and/or solutes present in a filtered fraction F coming out of a blood filtering device 1.

According to the invention, the process includes the steps of:
- having a blood filtering device 1 as described above;
- detecting a first electrical signal generated by the at least one front photodiode 66, 68 of the first sensor 6;
- detecting a second electrical signal generated by the at least one lateral photodiode 67, 69 of the first sensor 6;
- processing said first electrical signal and said second electrical signal to generate a signal indicative at least of the amount of suspended particles moving along the outlet conduit 5' of the filtering device 1.

As described above, processing the electrical signals generated by the front photodiode 66 and the lateral photodiode 67 makes it possible to detect the presence of red blood cells and thus to know the amount of blood in the filtrate fraction F.

Advantageously, the process for the detection of suspended particles and/or solutes comprises the following steps:
- processing said first electrical signal and said second electrical signal to generate a signal indicative of the quantity and/or type of solutes present in the filtrate fraction F at the output of the filtering device 1.

As described above, the processing of the electrical signals generated by the front photodiode 66 and by the lateral photodiode 67 also makes it possible to estimate the amount of particular solutes in the filtrate fraction F or in the dialysis fluid 4' leaving the filtering device 1, such as for example urea, or haemoglobin, or bilirubin.

Advantageously, the process for the detection of suspended particles and/or solutes comprises the following steps:
- comparing the signal indicative of the presence and/or quantity of the suspended particles with the signal indicative of the quantity and/or type of solutes present in the filtrate fraction F at the outlet of the filtering device 1;
- processing a signal indicative of the composition of the filtrate fraction F.

As described above, the comparison between the electrical signals generated by the two different photodiodes 66 and 67 allows to estimate the amount of various solutes present in the filtrate fraction F or in the dialysis fluid 4' leaving filter 2, such as urea, bilirubin, or haemoglobin dissolved in the filtrate fraction F for a hemodialysis effect.

Advantageously, the process for the detection of suspended particles and/or solutes comprises the following step:
- generating an alarm signal when the signal indicating at least the quantity of suspended particles in movement along the outlet conduit 5' exceeds a threshold value.

In practice it has been found that the blood filtering device, particularly for hemodialysis and/or haemofiltration apparatuses, according to the present invention, achieves the intended aim and objects as it is possible to monitor its integrity and performance in a highly sensitive and accurate manner.

Another advantage of the blood filtering device, according to the invention, consists in the fact of incorporating a "BLD" sensor - Blood Leak Detector - capable of generating an alarm signal in the presence of traces of blood in the filtrate fraction.

A further advantage consists in the fact that it is possible to distinguish, in the filtrate fraction, the presence of red blood cells from the presence of bilirubin and/or haemoglobin. In fact, it is generally the presence of red blood cells in the filtrate fraction that indicates that the filter has been damaged. Conversely, the detection of haemoglobin in the absence of red blood cells indicates the occurrence of a phenomenon of hemolysis which usually does not depend on the integrity of the filter.

Yet another advantage of the invention consists in the fact that the detection of traces of blood in the filtrate fraction is not affected by the surrounding ambient light conditions.

A further advantage of the filtering device, according to the invention, consists in the fact that the combination of the signals obtained from photodiodes working both in self-mix and in radiation absorption allows to verify the presence and estimate the quantity of different types of solutes present in the filtrate fraction at the output of the filtering device.

In particular, the possibility of estimating the presence of urea, moreover with the same sensor adapted to work as a BLD, makes it possible to know in real time the effectiveness of the therapeutic treatment, for example of hemodialysis, which is being carried out, being able to intervene accordingly, for example by interrupting or prolonging the therapeutic session when a desired purification of the blood is found, or by modifying the dialysis parameters during the session itself.

Yet another advantage of the invention consists in the fact that the combination of the signals obtained from photodiodes working both in self-mix and in radiation absorption allows obtaining very robust and accurate information about the presence of blood and/or solutes in the filtrate fraction.

A further advantage of the invention consists in the fact that it is inexpensive to manufacture and to fit into hemodialysis and/or haemofiltration apparatuses of known type.

## Claims

1. Blood filtering device (1), particularly for hemodialysis and/or haemofiltration apparatuses, comprising a filter (2) comprising a first compartment (30), adapted to allow the passage of blood (3, 3'), and a second compartment (40), said first compartment (30) and said second compartment (40) being separated by a membrane (20) adapted to allow the passage of a filtered fraction (F) from said first compartment (30) to said second compartment (40), said filtering device (1) comprising an outlet conduit (5') adapted to collect said filtered fraction (F) leaving said filter (2), said filtered fraction (F) flowing along said outlet conduit (5') along a flow direction (S), **characterized in that** said blood filtering device (1) comprises a first sensor (6) comprising:
- at least one semiconductor laser source (60, 61) comprising a laser cavity (62) and adapted to generate a laser light beam (64) which strikes said outlet conduit (5') along an irradiation direction (R) incident to said flow direction (S);
- at least one front photodiode (66, 68) placed along said irradiation direction (R) on a side opposite to said semiconductor laser source (60, 61) with respect to said outlet conduit (5'),
- at least one lateral photodiode (67, 69) placed along a diffusion direction (D) substantially orthogonal to said irradiation direction (R),
at least in correspondence of said semiconductor laser source (60, 61), of said at least one front photodiode (66, 68) and of said at least one lateral photodiode (67, 69), said outlet conduit (5') being transparent to said laser light beam (64),
said at least one front photodiode (66, 68) generating a first electrical signal dependent on the modulation of the power of said laser light beam (64) operated, according to a retro-injection interferometry effect, by suspended particles present within said filtered fraction (F) and moving along said outlet conduit (5'),
said at least one lateral photodiode (67, 69) generating a second electrical signal dependent on the part (65) of said laser light beam (64) which is diffused by said filtered fraction (F) along substantially said diffusion direction (D);
said filtering device (1) comprising a processing and control unit (8) programmed to process said first electrical signal and said second electrical signal to generate a signal indicative at least of the quantity of said suspended particles moving along said outlet conduit (5').

2. Blood filtering device (1), according to claim 1, **characterized in that** said processing and control unit (8) is programmed to process said first electrical signal and said second electrical signal to generate a signal indicative of the type and/or the quantity of solutes present in said filtrate fraction (F).

3. Filtering device (1), according to claim 2, **characterized in that** said processing and control unit (8) comprises a programmable memory (80) configured to receive and store at least one reference signal associated with at least one type of solute present in a reference liquid, said processing and control unit (8) being programmed to generate a signal indicative of the amount of said at least one type of solute present in said filtrate fraction (F) on the basis of a comparison with said signal reference associated with said at least one type of solute stored in said programmable memory (80) .

4. Blood filtering device (1), according to claim 1 or 2 or 3, **characterized in that** said filter (2) is a dialyzer filter comprising said first compartment (30), adapted to allow the passage of blood (3, 3'), and said second compartment (40), wherein said second compartment (40) is adapted to allow the passage of a dialysis liquid (4, 4'), said first compartment (30) and said second compartment (40) being separated by a semipermeable membrane (20) selective upon the crossing of said filtrate fraction (F) from said blood (3, 3') to said dialysis liquid (4, 4'), said outlet conduit (5') being adapted to collect a mixture (4') of said filtrate fraction (F) and said dialysis liquid leaving said dialyzer filter (2), wherein said mixture (4') of said filtrate fraction (F) and of said dialysis liquid flows along said outlet conduit (5') according to a flow direction (S),
said first electrical signal generated by said at least one front photodiode (66, 68) depending on the modulation of the power of said laser light beam (64) operated, according to a retro-injection interferometry effect, by suspended particles present inside said mixture (4') of said filtrate fraction (F) and said dialysis liquid and moving along said outlet conduit (5');
said second electrical signal generated by said at least one lateral photodiode (67, 69) depending on the part (65) of said laser light beam (64) which is diffused by said mixture (4') of said filtrate fraction (F) and of said dialysis liquid along substantially said diffusion direction (D).

5. Filtering device (1), according to claim 4, **characterized by** the fact that said processing and control unit (8) is programmed to process said first electrical signal, generated by said at least one front photodiode (66, 68), and said second electrical signal, generated by said at least one lateral photodiode (67, 69), to generate a signal indicative of the quantity of urea present in said mixture (4') of said filtrate fraction (F) and of said dialysis liquid.

6. Filtering device (1), according to claim 5, **characterized by** the fact that said processing and control unit (8) is programmed to execute a classifying algorithm of one or more features of said first electrical signal and one or more features of said second electrical signal, and to generate said signal indicative of the amount of urea present in said mixture (4') of said filtrate fraction (F) and of said dialysis liquid on the basis of said classifying algorithm.

7. Filtering device (1), according to one or more of claims 4 to 6, **characterized by** the fact of comprising an inlet conduit (5) adapted to convey said dialysis liquid (4) inlet to said dialyzer filter (2), said dialysis liquid (4) flowing along said inlet conduit (5) according to a flow direction (S), said filtering device (1) comprising a second sensor (9) comprising:
- at least one semiconductor laser source (90) comprising a laser cavity (92) and adapted to generate a laser light beam (94) which strikes said inlet conduit (5) along an irradiation direction incident to said flow direction (S);
- at least one front photodiode (96) placed along said irradiation direction on a side opposite to said semiconductor laser source (90) with respect to said inlet conduit (5),
- at least one lateral photodiode (97) placed along said diffusion direction (D) substantially orthogonal to said irradiation direction (R),
at least in correspondence of said second semiconductor laser source (90), of said at least one front photodiode (96) and of said at least one lateral photodiode (97), said inlet conduit (5) being transparent to said laser light beam (94),
said at least one front photodiode (96) generating an electrical signal dependent on the modulation of the power of said laser light beam (94) operated, according to a retro-injection interferometry effect, by suspended particles present inside said dialysis liquid (4) and moving along said inlet conduit (5);
said at least one lateral photodiode (97) generating an electrical signal depending on the part of the laser light beam (94) which is diffused by said dialysis liquid (4) along substantially said diffusion direction (D),
said processing and control unit (8) being programmed to use said electrical signals generated by said at least one front photodiode (96) and by said at least one side photodiode (97) of said second sensor (9) in subtraction respectively of said first electrical signal generated by said at least one front photodiode (66, 68) of said first sensor (6) and said second electrical signal generated by said at least one lateral photodiode (67, 69) of said first sensor (6) to generate said signal indicative of at least the quantity of said suspended particles moving along said outlet conduit (5'), deprived of the disturbances common to said electrical signals.

8. Filtering device (1), according to one or more of the preceding claims, **characterized in that** said first sensor (6) comprises a first semiconductor laser source (60) and at least a further semiconductor laser source (61, 61) that is selectable between:
(i) at least a second semiconductor laser source (61) adapted to generate a laser light beam having a different wavelength with respect to the laser light beam generated by said first semiconductor laser source (60);
(ii) at least a radiation source (61') adapted to generate a radiation that strikes said outlet conduit (5') along an irradiation direction incident to said flow direction (S).

9. Filtering device (1), according to claim 8, **characterized in that** it comprises at least two front photodiodes (66, 68) placed along said irradiation direction (R) on a side respectively opposite to said first semiconductor laser source (60) and to said second semiconductor laser source (61) with respect to said outlet conduit (5'), and at least two lateral photodiodes (67, 69) placed along a diffusion direction (D) substantially orthogonal to said irradiation direction (R) .

10. Filtering device (1), according to one or more of the preceding claims, **characterized in that** it comprises at least one spectrophotometric sensor (7) comprising:
- a radiation source (70) adapted to generate a radiation (72) which strikes said outlet conduit (5') along a direction of radiation incident to said flow direction (S),
- a photodiode (74) placed along said irradiation direction on a side opposite to said radiation source (70) with respect to said outlet conduit (5'), said photodiode (74) generating an electrical signal indicative of the transmittance of said radiation through said filtrate fraction (F), said transmittance of said radiation (72) depending on the quantity and/or type of solutes present in said filtrate fraction (F),
said processing and control unit (8) being programmed to process said electrical signal to generate a signal indicative of the quantity and/or type of solutes present in said filtrate fraction (F).

11. Filtering device (1), according to one or more of the preceding claims, **characterized in that** said processing and control unit (8) is programmed to generate an alarm signal when said signal indicating at least the quantity of suspended particles in movement along said outlet conduit (5') exceeds a threshold value.

12. Hemodialysis and/or hemofiltration apparatus (100), **characterized in that** it comprises a blood filtering device (1) according to one or more of the preceding claims.

13. Process for detecting suspended particles and/or solutes present in a filtered fraction (F) coming out of a blood filtering device (1), **characterized by** the fact that it includes the following steps:
- having a blood filtering device (1) according to one or more of claims 1 to 11;
- detecting a first electrical signal generated by said at least one front photodiode (66, 68) of said first sensor (6);
- detecting a second electrical signal generated by said at least one lateral photodiode (67, 69) of said first sensor (6);
- processing said first electrical signal and said second electrical signal to generate a signal indicative at least of the amount of said suspended particles moving along said outlet conduit (5') of said filtering device (1) .

14. Process for the detection of suspended particles and/or solutes, according to claim 13, **characterized in that** it comprises the following step:
- processing said first electrical signal and said second electrical signal to generate a signal indicative of the quantity and/or type of solutes present in said filtrate fraction (F) at the output of said filtering device (1).

15. Process for the detection of suspended particles and/or solutes, according to claim 14, **characterized in that** it comprises the following steps:
- comparing said signal indicative of the presence and/or quantity of said suspended particles with said signal indicative of the quantity and/or type of solutes present in said filtrate fraction (F) at the outlet of said filtering device (1);
- generating a signal indicative of the composition of said filtrate fraction (F).

## Patentansprüche

1. Blutfiltervorrichtung (1), insbesondere für Hämodialyse- und/oder Hämofiltrationsgeräte, mit einem Filter (2), der einen ersten Abschnitt (30), der so eingerichtet ist, dass er den Blutdurchgang (3, 3') ermöglicht, und einen zweiten Abschnitt (40) umfasst, wobei der erste Abschnitt (30) und der zweite Abschnitt (40) durch eine Membran (20) getrennt sind, die so eingerichtet ist, dass sie den Durchgang einer gefilterten Fraktion (F) von dem ersten Abschnitt (30) zu dem zweiten Abschnitt (40) ermöglicht, wobei die Filtervorrichtung (1) eine Auslassleitung (5') umfasst, die eingerichtet ist, die gefilterte Fraktion (F), die den Filter (2) verlässt, zu sammeln, wobei die gefilterte Fraktion (F) entlang der Auslassleitung (5') entlang einer Strömungsrichtung (S) fließt, **dadurch gekennzeichnet, dass** die Blutfiltervorrichtung (1) einen ersten Sensor (6) umfasst, umfassend:
- mindestens eine Halbleiterlaserquelle (60, 61), die eine Laserkavität (62) umfasst und eingerichtet ist, einen Laserlichtstrahl (64) zu erzeugen, der auf die Auslassleitung (5') entlang einer Bestrahlungsrichtung (R) auftrifft, die der Strömungsrichtung (S) einfallend ist;
- mindestens eine vordere Fotodiode (66, 68), die entlang der Bestrahlungsrichtung (R) auf einer der Halbleiterlaserquelle (60, 61) gegenüberliegenden Seite in Bezug auf die Auslassleitung (5') angeordnet ist,
- mindestens eine seitliche Fotodiode (67, 69), die entlang einer Streuungsrichtung (D) angeordnet ist, die im Wesentlichen orthogonal zu der Bestrahlungsrichtung (R) verläuft,
zumindest in Übereinstimmung mit der Halbleiterlaserquelle (60, 61), der mindestens einen vorderen Fotodiode (66, 68) und der mindestens einen seitlichen Fotodiode (67, 69), wobei die Aulassleitung (5') für den Laserlichtstrahl (64) transparent ist,
wobei die mindestens eine vordere Fotodiode (66, 68) ein erstes elektrisches Signal erzeugt, das von der Modulation der Leistung des Laserlichtstrahls (64) abhängt, der gemäß einem Rückinjektions-Interferometrie-Effekt durch suspendierte Partikel, die in der gefilterten Fraktion (F) vorhanden sind und sich entlang der Auslassleitung (5') bewegen, betrieben wird,
wobei die mindestens eine seitliche Fotodiode (67, 69) ein zweites elektrisches Signal erzeugt, das von dem Teil (65) des Laserlichtstrahls (64) abhängt, der von der gefilterten Fraktion (F) im Wesentlichen entlang der Streuungsrichtung (D) gestreut wird;
wobei die Filtervorrichtung (1) eine Verarbeitungs- und Steuereinheit (8) umfasst, die so programmiert ist, dass sie das erste elektrische Signal und das zweite elektrische Signal verarbeitet, um ein Signal zu erzeugen, das zumindest die Menge der sich entlang der Auslassleitung (5') suspendierten Partikel anzeigt.

2. Blutfiltervorrichtung (1) nach Anspruch 1, **dadurch gekennzeichnet, dass** die Verarbeitungs- und Steuereinheit (8) so programmiert ist, dass sie das erste elektrische Signal und das zweite elektrische Signal verarbeitet, um ein Signal zu erzeugen, das die Art und/oder die Menge der in der gefilterten Fraktion (F) vorhandenen gelösten Substanzen anzeigt.

3. Filtervorrichtung (1) nach Anspruch 2, **dadurch gekennzeichnet, dass** die Verarbeitungs- und Steuereinheit (8) einen programmierbaren Speicher (80) umfasst, der so ausgebildet ist, dass er mindestens ein Referenzsignal empfängt und speichert, das mindestens einem Typ einer gelösten Substanz zugeordnet ist, der in einer Referenzflüssigkeit vorhanden ist, wobei die Verarbeitungs- und Steuereinheit (8) so programmiert ist, dass sie ein Signal erzeugt, das die Menge des mindestens einen Typs der gelösten Substanz, der in der gefilterten Fraktion (F) vorhanden ist, auf der Grundlage eines Vergleichs mit dem Referenzsignal anzeigt, das dem mindestens einen Typ der gelösten Substanz zugeordnet ist, der in dem programmierbaren Speicher (80) gespeichert ist

4. Blutfiltervorrichtung (1) nach Anspruch 1 oder 2 oder 3, **dadurch gekennzeichnet, dass** der Filter (2) ein Dialysatorfilter ist, der der erste Abschnitt (30), der den Blutdurchgang (3, 3') ermöglicht, und der zweite Abschnitt (40), wobei der zweite Abschnitt (40) so eingerichtet ist, dass er den Durchgang einer Dialyseflüssigkeit (4, 4') ermöglicht, wobei der erste Abschnitt (30) und der zweite Abschnitt (40) durch eine halbdurchlässige Membran (20) getrennt sind, die beim Übergang der gefilterten Fraktion (F) aus dem Blut (3, 3') zu der Dialyseflüssigkeit (4, 4') selektiv ist, wobei die Auslassleitung (5') eingerichtet ist, um eine Mischung (4') der gefilterten Fraktion (F) und der Dialyseflüssigkeit, die den Dialysatorfilter (2) verlässt, zu sammeln, wobei die Mischung (4') der gefilterten Fraktion (F) und der Dialyseflüssigkeit entlang der Auslassleitung (5') gemäß einer Strömungsrichtung (S) fließt,
wobei das erste elektrische Signal, das von der mindestens einen vorderen Fotodiode (66, 68) erzeugt wird, von der Modulation der Leistung des Laserlichtstrahls (64) abhängt, der gemäß einem Rückinjektions-Interferometrie-Effekt durch suspendierte Partikel, die in der Mischung (4') der gefilterten Fraktion (F) und der Dialyseflüssigkeit vorhanden sind und sich entlang der Auslassleitung (5') bewegen, betrieben wird;
wobei das zweite elektrische Signal, das von der mindestens einen seitlichen Fotodiode (67, 69) erzeugt wird, von dem Teil (65) des Laserlichtstrahls (64) abhängt, der von der Mischung (4') der gefilterten Fraktion (F) und der Dialyseflüssigkeit im Wesentlichen entlang der Streuungsrichtung (D) gestreut wird.

5. Filtervorrichtung (1) nach Anspruch 4, **dadurch gekennzeichnet, dass** die Verarbeitungs- und Steuereinheit (8) so programmiert ist, dass sie das erste elektrische Signal, das von der mindestens einen vorderen Fotodiode (66, 68) erzeugt wird, und das zweite elektrische Signal, das von der mindestens einen seitlichen Fotodiode (67, 69) erzeugt wird, verarbeitet, um ein Signal zu erzeugen, das die Menge an Harnstoff anzeigt, die in der Mischung (4') der gefilterten Fraktion (F) und der Dialyseflüssigkeit vorhanden ist.

6. Filtervorrichtung (1) nach Anspruch 5, **dadurch gekennzeichnet, dass** die Verarbeitungs- und Steuereinheit (8) so programmiert ist, dass sie einen Klassifizierungsalgorithmus bestehend aus einem oder mehreren Merkmalen des ersten elektrischen Signals und einem oder mehreren Merkmalen des zweiten elektrischen Signals ausführt und das Signal, das die Menge des in der Mischung (4') der gefilterten Fraktion (F) und der Dialyseflüssigkeit vorhandenen Harnstoffs anzeigt, auf der Grundlage des Klassifizierungsalgorithmus erzeugt.

7. Filtervorrichtung (1) nach einem oder mehreren der Ansprüche 4 bis 6, **dadurch gekennzeichnet, dass** sie eine Einlassleitung (5) umfasst, die so eingerichtet ist, dass sie die Dialyseflüssigkeit (4) zum Einlass des Dialysatorfilters (2) befördert, wobei die Dialyseflüssigkeit (4) entlang der Einlassleitung (5) gemäß einer Strömungsrichtung (S) fließt, wobei die Filtervorrichtung (1) einen zweiten Sensor (9) umfasst, umfassend:
mindestens eine Halbleiterlaserquelle (90), die eine Laserkavität (92) aufweist und eingerichtet ist, einen Laserlichtstrahl (94) zu erzeugen, der auf die Einlassleitung (5) entlang einer Bestrahlungsrichtung auftrifft, die der Strömungsrichtung (S) einfallend ist;
- mindestens eine vordere Fotodiode (96), die entlang der Bestrahlungsrichtung auf einer der Halbleiterlaserquelle (90) gegenüberliegenden Seite in Bezug auf die Einlassleitung (5) angeordnet ist,
- mindestens eine seitliche Fotodiode (97), die entlang der Streuungsrichtung (D) im Wesentlichen orthogonal zur Bestrahlungsrichtung (R) angeordnet ist,
zumindest in Übereinstimmung mit der zweiten Halbleiterlaserquelle (90) der mindestens einen vorderen Fotodiode (96) und der mindestens einen seitlichen Fotodiode (97), wobei die Einlassleitung (5) für den Laserlichtstrahl (94) transparent ist, wobei die mindestens eine vordere Fotodiode (96) ein elektrisches Signal erzeugt, das von der Modulation der Leistung des Laserlichtstrahls (94) abhängt, der gemäß einem Rückinjektions-Interferometrie-Effekt durch suspendierte Partikel betrieben wird, die in der Dialyseflüssigkeit (4) vorhanden sind und sich entlang der Einlassleitung (5) bewegen;
wobei die mindestens eine seitliche Fotodiode (97) ein elektrisches Signal erzeugt, das von dem Teil des Laserlichtstrahls (94) abhängt, der von der Dialyseflüssigkeit (4) im Wesentlichen entlang der Streuungsrichtung (D) gestreut wird,
wobei die Verarbeitungs- und Steuereinheit (8) so programmiert ist, dass sie die elektrischen Signale, die von der mindestens einen vorderen Fotodiode (96) und der mindestens einen seitlichen Fotodiode (97) des zweiten Sensors (9) erzeugt werden zur Subtraktion des ersten elektrischen Signals, das durch der mindestens einen vorderen Fotodiode (66, 68) des ersten Sensors erzeugt wird und des zweiten elektrischen Signals, das durch die mindestens eine seitliche Fotodiode (67, 69) des ersten Sensors (6) erzeugt wird, verwendet, um das Signal zu erzeugen, das zumindest die Menge der suspendierten Partikel anzeigt, die sich entlang der Auslassleitung (5') bewegen, wobei die den elektrischen Signalen gemeinsamen Störungen entfernt werden.

8. Filtervorrichtung (1) nach einem oder mehreren der vorhergehenden Ansprüche, **dadurch gekennzeichnet, dass** der erste Sensor (6) eine erste Halbleiterlaserquelle (60) und mindestens eine weitere Halbleiterlaserquelle (61, 61) umfasst, die zwischen folgenden ausgewählt werden kann:
(i) mindestens einer zweiten Halbleiterlaserquelle (61), die so eingerichtet ist, dass sie einen Laserlichtstrahl erzeugt, der eine andere Wellenlänge als der von der ersten Halbleiterlaserquelle (6O) erzeugte Laserlichtstrahl aufweist;
(ii) mindestens einer Strahlungsquelle (61'), die eingerichtet ist, eine Strahlung zu erzeugen, die auf die Auslassleitung (5') entlang einer Bestrahlungsrichtung auftrifft, die der Strömungsrichtung (S) einfallend ist

9. Filtervorrichtung (1) nach Anspruch 8, **dadurch gekennzeichnet, dass** sie mindestens zwei vordere Fotodioden (66, 68) umfasst, die entlang der Bestrahlungsrichtung (R) auf einer Seite angeordnet sind, die der ersten Halbleiterlaserquelle (60) bzw. der zweiten Halbleiterlaserquelle (61) in Bezug auf die Auslassleitung (5') gegenüberliegt, und mindestens zwei seitliche Fotodioden (67, 69), die entlang einer Streuungsrichtung (D) angeordnet sind, die im Wesentlichen orthogonal zur Bestrahlungsrichtung (R) ist.

10. Filtervorrichtung (1) nach einem oder mehreren der vorhergehenden Ansprüche, **dadurch gekennzeichnet, dass** sie mindestens einen spektrophotometrischen Sensor (7) umfassend:
- eine Strahlungsquelle (70), die eingerichtet ist, eine Strahlung (72) zu erzeugen, die auf die Auslassleitung (5') entlang einer Strahlungsrichtung auftrifft, die der Strömungsrichtung (S) einfallend ist,
- eine Fotodiode (74), die entlang der Bestrahlungsrichtung auf einer der Strahlungsquelle (70) gegenüberliegenden Seite in Bezug auf die Auslassleitung (5') angeordnet ist, wobei die Fotodiode (74) ein elektrisches Signal erzeugt, das die Durchlässigkeit der Strahlung durch die gefilterte Fraktion (F) anzeigt, wobei die Durchlässigkeit der Strahlung (72) von der Menge und/oder der Art der in der gefilterten Fraktion (F) vorhandenen gelösten Substanzen abhängt, wobei die Verarbeitungs- und Steuereinheit (8) so programmiert ist, dass sie das elektrische Signal verarbeitet, um ein Signal zu erzeugen, das die Menge und/oder die Art der in der gefilterten Fraktion (F) vorhandenen gelösten Substanzen anzeigt.

11. Filtervorrichtung (1) nach einem oder mehreren der vorhergehenden Ansprüche, **dadurch gekennzeichnet, dass** die Verarbeitungs- und Steuereinheit (8) so programmiert ist, dass sie ein Alarmsignal erzeugt, wenn das Signal, das zumindest die Menge an suspendierten Partikeln in Bewegung entlang der Auslassleitung (5') anzeigt, einen Schwellenwert überschreitet.

12. Hämodialyse- und/oder Hämofiltrationsgerät (100), **dadurch gekennzeichnet, dass** es eine Blutfiltervorrichtung (1) nach einem oder mehreren der vorhergehenden Ansprüche umfasst.

13. Verfahren zur Erfassung von suspendierten Partikeln und/oder gelösten Substanzen, die in einer gefilterten Fraktion (F) vorhanden sind, die aus einer Blutfiltervorrichtung (1) kommt, **dadurch gekennzeichnet, dass** es die folgenden Schritte umfasst:
- Aufweisen einer Blutfiltervorrichtung (1) nach einem oder mehreren der Ansprüche 1 bis 11;
- Erfassen eines ersten elektrischen Signals, das von der mindestens einen vorderen Fotodiode (66, 68) des ersten Sensors (6) erzeugt wird;
- Erfassen eines zweiten elektrischen Signals, das von der mindestens einen seitlichen Fotodiode (67, 69) des ersten Sensors (6) erzeugt wird;
- Verarbeiten des ersten elektrischen Signals und des zweiten elektrischen Signals, um ein Signal zu erzeugen, das zumindest die Menge der suspendierten Partikeln anzeigt, die sich entlang der Auslassleitung (5') der Filtervorrichtung (1) bewegen.

14. Verfahren zur Erfassung von suspendierten Partikeln und/oder gelösten Substanzen nach Anspruch 13, **dadurch gekennzeichnet, dass** es den folgenden Schritt umfasst:
- Verarbeiten des ersten elektrischen Signals und des zweiten elektrischen Signals, um ein Signal zu erzeugen, das die Menge und/oder die Art der gelösten Substanzen anzeigt, die in der gefilterten Fraktion (F) am Ausgang der Filtervorrichtung (1) vorhanden sind

15. Verfahren zur Erfassung von suspendierten Partikeln und/oder gelösten Substanzen nach Anspruch 14, **dadurch gekennzeichnet, dass** es die folgenden Schritte umfasst:
- Vergleichen des Signals, das das Vorhandensein und/oder die Menge der suspendierten Partikel anzeigt, mit dem Signal, das die Menge und/oder die Art der gelösten Substanzen anzeigt, die in der gefilterten Fraktion (F) am Ausgang der Filtervorrichtung (1) vorhanden sind;
- Erzeugen eines Signals, das die Zusammensetzung der gefilterten Fraktion (F) anzeigt.

## Revendications

1. Dispositif de filtrage de sang (1), en particulier pour des appareils d'hémodialyse et/ou d'hémofiltration, comprenant un filtre (2) comprenant un premier compartiment (30), adapté pour permettre le passage de sang (3, 3'), et un second compartiment (40), ledit premier compartiment (30) et ledit second compartiment (40) étant séparés par une membrane (20) adaptée pour permettre le passage d'une fraction filtrée (F) dudit premier compartiment (30) vers ledit second compartiment (40), ledit dispositif de filtrage (1) comprenant un conduit de sortie (5') adapté pour collecter ladite fraction filtrée (F) sortant dudit filtre (2), ladite fraction filtrée (F) s'écoulant le long dudit conduit de sortie (5') le long d'une direction d'écoulement (S), **caractérisé en ce que** ledit dispositif de filtrage de sang (1) comprend un premier capteur (6) comprenant:
- au moins une source laser à semi-conducteur (60, 61) comprenant une cavité laser (62) et adaptée pour générer un faisceau de lumière laser (64) qui frappe ledit conduit de sortie (5') le long d'une direction d'irradiation (R) incidente par rapport à ladite direction d'écoulement (S);
- au moins une photodiode frontale (66, 68) placée le long de ladite direction d'irradiation (R) sur un côté opposé à ladite source laser à semi-conducteur (60, 61) par rapport audit conduit de sortie (5'),
- au moins une photodiode latérale (67, 69) placée le long d'une direction de diffusion (D) sensiblement orthogonale à ladite direction d'irradiation (R),
au moins en correspondance de ladite source laser à semi-conducteur (60, 61), de ladite au moins une photodiode frontale (66, 68) et de ladite au moins une photodiode latérale (67, 69), ledit conduit de sortie (5') étant transparent audit faisceau de lumière laser (64),
ladite au moins une photodiode frontale (66,68) générant un premier signal électrique dépendant de la modulation de la puissance dudit faisceau de lumière laser (64) opérée, selon un effet d'interférométrie à rétro-injection, par les particules en suspension présentes dans ladite fraction filtrée (F) et se déplaçant le long dudit conduit de sortie (5'),
ladite au moins une photodiode latérale (67, 69) générant un second signal électrique dépendant de la partie (65) dudit faisceau de lumière laser (64) qui est diffusée par ladite fraction filtrée (F) sensiblement le long de ladite direction de diffusion (D);
ledit dispositif de filtrage (1) comprenant une unité de traitement et de contrôle (8) programmée pour traiter ledit premier signal électrique et ledit second signal électrique afin de générer un signal indiquant au moins la quantité desdites particules en suspension se déplaçant le long dudit conduit de sortie (5').

2. Dispositif de filtrage de sang (1), selon la revendication 1, **caractérisé en ce que** ladite unité de traitement et de contrôle (8) est programmée pour traiter ledit premier signal électrique et ledit second signal électrique afin de générer un signal indiquant le type et/ou la quantité de solutés présents dans ladite fraction de filtrat (F).

3. Dispositif de filtrage (1), selon la revendication 2, **caractérisé en ce que** ladite unité de traitement et de contrôle (8) comprend une mémoire programmable (80) configurée pour recevoir et stocker au moins un signal de référence associé à au moins un type de soluté présent dans un liquide de référence, ladite unité de traitement et de contrôle (8) étant programmée pour générer un signal indicatif de la quantité dudit au moins un type de soluté présent dans ladite fraction de filtrat (F) sur la base d'une comparaison avec ledit signal de référence associé audit au moins un type de soluté stocké dans ladite mémoire programmable (80).

4. Dispositif de filtrage de sang (1), selon la revendication 1 ou 2 ou 3, **caractérisé en ce que** ledit filtre (2) est un filtre dialyseur comprenant ledit premier compartiment (30), adapté pour permettre le passage du sang (3, 3'), et ledit second compartiment (40), dans lequel ledit second compartiment (40) est adapté pour permettre le passage d'un liquide de dialyse (4, 4'), ledit premier compartiment (3O) et ledit second compartiment (40) étant séparés par une membrane semi-perméable (20) sélective au passage de ladite fraction de filtrat (F) dudit sang (3, 3') vers ledit liquide de dialyse (4, 4'), ledit conduit de sortie (5') étant adapté pour collecter un mélange (4') de ladite fraction de filtrat (F) et dudit liquide de dialyse quittant ledit filtre dialyseur (2), dans lequel ledit mélange (4') de ladite fraction de filtrat (F) et dudit liquide de dialyse s'écoule le long dudit conduit de sortie (5') selon une direction d'écoulement (S),
ledit premier signal électrique généré par ladite au moins une photodiode frontale (66, 68) en fonction de la modulation de la puissance dudit faisceau de lumière laser (64) opérée, selon un effet d'interférométrie à rétro-injection, par les particules en suspension présentes à l'intérieur dudit mélange (4') de ladite fraction de filtrat (F) et dudit liquide de dialyse et se déplaçant le long dudit conduit de sortie (5');
ledit second signal électrique généré par ladite au moins une photodiode latérale (67, 69) en fonction de la partie (65) dudit faisceau de lumière laser (64) qui est diffusée par ledit mélange (4') de ladite fraction de filtrat (F) et dudit liquide de dialyse le long de sensiblement ladite direction de diffusion (D).

5. Dispositif de filtrage (1), selon la revendication 4, **caractérisé par le fait que** ladite unité de traitement et de contrôle (8) est programmée pour traiter ledit premier signal électrique, généré par ladite au moins une photodiode frontale (66, 68), et ledit second signal électrique, généré par ladite au moins une photodiode latérale (67, 69), pour générer un signal indicatif de la quantité d'urée présente dans ledit mélange (4') de ladite fraction de filtrat (F) et dudit liquide de dialyse.

6. Dispositif de filtrage (1), selon la revendication 5, **caractérisé par le fait que** ladite unité de traitement et de contrôle (8) est programmée pour exécuter un algorithme de classification d'une ou plusieurs caractéristiques dudit premier signal électrique et d'une ou plusieurs caractéristiques dudit second signal électrique, et pour générer ledit signal indicatif de la quantité d'urée présente dans ledit mélange (4') de ladite fraction de filtrat (F) et dudit liquide de dialyse sur la base dudit algorithme de classification.

7. Dispositif de filtrage (1), selon une ou plusieurs des revendications 4 à 6, **caractérisé par le fait qu'**il comprend un conduit d'entrée (5) adapté pour acheminer ledit liquide de dialyse (4) en entrée dudit filtre dialyseur (2), ledit liquide de dialyse (4) s'écoulant le long dudit conduit d'entrée (5) selon une direction d'écoulement (S), ledit dispositif de filtrage (1) comprenant un second capteur (9) comprenant:
au moins une source laser à semi-conducteur (9O) comprenant une cavité laser (92) et adaptée pour générer un faisceau de lumière laser (94) qui frappe ledit conduit d'entrée (5) le long d'une direction d'irradiation incidente par rapport à ladite direction d'écoulement (S);
- au moins une photodiode frontale (96) placée le long de ladite direction d'irradiation sur un côté opposé à ladite source laser à semi-conducteur (9O) par rapport audit conduit d'entrée (5),
- au moins une photodiode latérale (97) placée le long de ladite direction de diffusion (D), sensiblement orthogonale à ladite direction d'irradiation (R),
au moins en correspondance de ladite seconde source laser à semi-conducteur (9O), de ladite au moins une photodiode frontale (96) et de ladite au moins une photodiode latérale (97), ledit conduit d'entrée (5) étant transparent audit faisceau de lumière laser (94), ladite au moins une photodiode frontale (96) générant un signal électrique dépendant de la modulation de la puissance dudit faisceau de lumière laser (94) opérée, selon un effet d'interférométrie à rétro-injection, par les particules en suspension présentes à l'intérieur dudit liquide de dialyse (4) et se déplaçant le long dudit conduit d'entrée (5);
ladite au moins une photodiode latérale (97) génère un signal électrique en fonction de la partie du faisceau de lumière laser (94) qui est diffusée par ledit liquide de dialyse (4) sensiblement le long de ladite direction de diffusion (D),
ladite unité de traitement et de contrôle (8) étant programmée pour utiliser lesdits signaux électriques générés par ladite au moins une photodiode frontale (96) et par ladite au moins une photodiode latérale (97) dudit second capteur (9) en soustraction respectivement dudit premier signal électrique généré par ladite au moins une photodiode frontale (66, 68) dudit premier capteur (6) et dudit second signal électrique généré par ladite au moins une photodiode latérale (67, 69) dudit premier capteur (6) pour générer ledit signal indicatif d'au moins la quantité desdites particules en suspension se déplaçant le long dudit conduit de sortie (5'), dépourvu des perturbations communes auxdits signaux électriques.

8. Dispositif de filtrage (1), selon l'une ou plusieurs des revendications précédentes, **caractérisé en ce que** ledit premier capteur (6) comprend une première source laser à semi-conducteur (60) et au moins une autre source laser à semi-conducteur (61, 61) qui peut être sélectionnée entre:
(i) au moins une seconde source laser à semi-conducteur (61) adaptée pour générer un faisceau de lumière laser ayant une longueur d'onde différente de celle du faisceau de lumière laser généré par ladite première source laser à semi-conducteur (6O);
(ii) au moins une source de rayonnement (61') adaptée pour générer un rayonnement qui frappe ledit conduit de sortie (5') le long d'une direction d'irradiation incidente par rapport à ladite direction d'écoulement (S)

9. Dispositif de filtrage (1), selon la revendication 8, **caractérisé en ce qu'**il comprend au moins deux photodiodes frontales (66, 68) placées le long de ladite direction d'irradiation (R) sur un côté respectivement opposé à ladite première source laser à semi-conducteur (60) et à ladite seconde source laser à semi-conducteur (61) par rapport audit conduit de sortie (5'), et au moins deux photodiodes latérales (67, 69) placées le long d'une direction de diffusion (D) sensiblement orthogonale à ladite direction d'irradiation (R).

10. Dispositif de filtrage (1), selon l'une ou plusieurs des revendications précédentes, **caractérisé en ce qu'**il comprend au moins un capteur spectrophotométrique (7) comprenant:
- une source de rayonnement (70) adaptée pour générer un rayonnement (72) qui frappe ledit conduit de sortie (5') le long d'une direction de rayonnement incidente par rapport à ladite direction d'écoulement (S),
- une photodiode (74) placée le long de ladite direction d'irradiation sur un côté opposé à ladite source de rayonnement (70) par rapport audit conduit de sortie (5'), ladite photodiode (74) générant un signal électrique indicatif de la transmittance dudit rayonnement à travers ladite fraction de filtrat (F), ladite transmittance dudit rayonnement (72) dépendant de la quantité et/ou du type de solutés présents dans ladite fraction de filtrat (F), ladite unité de traitement et de contrôle (8) étant programmée pour traiter ledit signal électrique afin de générer un signal indiquant la quantité et/ou le type de solutés présents dans ladite fraction de filtrat (F).

11. Dispositif de filtrage (1), selon l'une ou plusieurs des revendications précédentes, **caractérisé en ce que** ladite unité de traitement et de contrôle (8) est programmée pour générer un signal d'alarme lorsque ledit signal indiquant au moins la quantité de particules en suspension en mouvement le long dudit conduit de sortie (5') dépasse une valeur seuil.

12. Appareil d'hémodialyse et/ou d'hémofiltration (100), **caractérisé en ce qu'**il comprend un dispositif de filtrage de sang (1) selon l'une ou plusieurs des revendications précédentes.

13. Procédé de détection des particules en suspension et/ou des solutés présents dans une fraction filtrée (F) sortant d'un dispositif de filtrage de sang (1), **caractérisé par le fait qu'**il comprend les étapes suivantes:
- avoir un dispositif de filtrage de sang (1) selon l'une ou plusieurs des revendications 1 à 11;
- détecter un premier signal électrique généré par ladite au moins une photodiode frontale (66, 68) dudit premier capteur (6);
- détecter un second signal électrique généré par ladite au moins une photodiode latérale (67, 69) dudit premier capteur (6);
- traiter ledit premier signal électrique et ledit second signal électrique pour générer un signal indiquant au moins la quantité de particules en suspension se déplaçant le long dudit conduit de sortie (5') dudit dispositif de filtrage (1).

14. Procédé de détection de particules en suspension et/ou de solutés, selon la revendication 13, **caractérisé en ce qu'**il comprend l'étape suivante:
- traiter ledit premier signal électrique et ledit second signal électrique pour générer un signal indiquant la quantité et/ou le type de solutés présents dans ladite fraction de filtrat (F) à la sortie dudit dispositif de filtrage (1)

15. Procédé de détection de particules en suspension et/ou de solutés, selon la revendication 14, **caractérisé en ce qu'**il comprend l'étape suivante:
- comparer ledit signal indiquant la présence et/ou la quantité desdites particules en suspension avec ledit signal indiquant la quantité et/ou le type de solutés présents dans ladite fraction de filtrat (F) à la sortie dudit dispositif de filtrage (1);
- générer un signal indiquant la composition de ladite fraction de filtrat (F).
